# EUROPEAN PATENT APPLICATION

(11) **EP 4 696 289 A2**
(43) Date of publication of application: **18.02.2026**
(21) Application number: 25226009.6
(22) Date of filing: 03.03.2023
(51) Int. Cl.: A61F 7/02

(54) **NIGHTTIME WARMING BLANKET**

(30) Priority: 03.03.2022 CN 202210201110; 02.03.2023 US 202318116562
(62) Divisional of application: 23714155.1
(71) Applicant: NANHAI NANXIN NON-WOVEN CO. LTD., Jiojiang Town, Nanhai District Foshan, Guangdong 528200 (CN)
(72) Inventor: WU, Dong, Jiangsu, 215526 (CN); JIN, Yongji, Jiangsu, 215526 (CN); ZHAN, Weiqin, Jiangsu, 215526 (CN); FU, Linlei, Jiangsu, 215526 (CN)
(74) Representative: Meissner Bolte Nürnberg

(57) **Abstract**

Warming blankets are provided, in which the warming blankets include (i) a perspiration absorptive layer (PAL); (ii) a metal coating layer (MCL); and (iii) a transparent coating layer (TCL); wherein the MCL is located directly or indirectly between the PAL and the TCL. Methods of producing a warming blanket are also provided.

## Description

This application claims priority under 35 U.S.C. §119 to Chinese Patent Application No. 202210201110.9 filed March 3, 2022, which is expressly incorporated by reference herein in its entirety.

Embodiments of the presently-disclosed invention relate generally to warming blankets, such as for nighttime use, including (i) a perspiration absorptive layer (PAL); (ii) a metal coating layer (MCL); and (iii) a transparent coating layer (TCL), in which the MCL is located directly or indirectly between the PAL and the TCL. Embodiments of the presently-disclosed invention also relate to methods of producing a warming blanket.

Metalized materials, such as metalized blankets, traditionally include a metal coating applied to a base substrate, such as a nonwoven or a film. Such metalized materials, for example, provide a mechanism to by which a user's body heat is significantly retained. In this regard, the metalized materials (e.g., also known as a space blanket, mylar blanket, first aid blanket, safety blanket, thermal blanket, etc.) include a heat-reflective metal coating applied to a thin plastic film or nonwoven. Ideally, the metalized materials reflect around 90% of a user's body heat to mitigate heat loss from the user's body.

One drawback of some metalized materials relates their lack of breathability and/or flexibility, as well as lack of tailoring for use in specific environments. In this regard, applications of such metalized blankets for the retention of body heat may also desire a desirable level of vapor permeability and/or flexibility (e.g., to easily conform to a user's body).

One or more embodiments of the invention may address one or more of the aforementioned problems. Certain embodiments according to the invention provide a warming blanket, such as for use outside and/or during the nighttime, including (i) a perspiration absorptive layer (PAL); (ii) a metal coating layer (MCL); and (iii) a transparent coating layer (TCL); wherein the MCL is located directly or indirectly between the PAL and the TCL.

In another aspect, the present invention provides a method of producing a method of producing a warming blanket, such as for use outside and/or during the nighttime, comprising the following: (i) providing a transparent coating layer (TCL); (ii) depositing a metal coating layer (MCL) directly onto the TCL; (iii) providing or forming a perspiration absorptive layer (PAL); and (iv) bonding the PAL to the MCL to provide the warming blanket.

### BRIEF DESCRIPTION OF THE DRAWING(S)

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. Like numbers refer to like elements throughout, and wherein:
Figure 1 illustrates a warming blanket in accordance with certain embodiments of the invention;
Figure 2 illustrates a perspiration absorptive layer (PAL) including a plurality of through-holes in accordance with certain embodiments of the invention; and
Figure 3 illustrates the PAL of Figure 2 overlying a metal coating layer (MCL) that is visible through the plurality of through-holes, in accordance with certain embodiments of the invention.

### DETAILED DESCRIPTION

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise.

Certain embodiments of the invention generally relate to warming blankets, such as for use outside and/or during the nighttime, that include a perspiration absorptive layer (PAL), a metal coating layer (MCL), and a transparent coating layer (TCL), in which the MCL is located directly or indirectly between the PAL and the TCL. For example, the TCL may define a first outermost layer of the warming blanket and the PAL may define a second outermost layer of the warming blanket, in which the MCL constitutes at least one layer between the two outermost layers of the warming blanket. The warming blanket, for example, may be particularly suitable for use as a warming system for outside activities in a cold environment and/or during the nighttime (e.g., after sunset). The TCL, for example, provides a layer that mostly transmits several wavelengths of light therethrough and enables the external light (e.g., electromagnetic radiation) to reach the MCL, while in accordance with certain embodiments the TCL also functions as a layer of thermal insulation to mitigate heat loss from the MCL into the external environment. The MCL layer, for example, provides thermal reflection that may reflect electromagnetic radiation from the external environment (e.g., back towards the external environment) and from the body of a user (e.g., back towards the user). The PAL, for example, may comprise a fabric that is capable of absorbing and/or wicking away perspiration from a user's body, which may be particularly desirable as any perspiration that remains on a user's body and/or is allowed to pool against a user's body will eventually cool-off and act as a heat-sink and undesirably pull heat from the body of the user. Additionally, the PAL may comprise a plurality of through-holes that function as windows or unobstructed gateways for electromagnetic radiation leaving a user to strike the MCL and be reflected back to the user to prevent or mitigate a loss of body heat from the user. In use, for example, the PAL may be positioned adjacent or proximate a user, such as a mammal, and the plurality of through-holes enable a high level of generally unobstructed access to the MCL by radiation or heat emitted by a user, in which this radiation or heat is mostly (or all) reflected back to the user by the MCL. In use, that is, the PAL will typically be positioned proximate a user, while the TCL will be positioned distal from the user.

In accordance with certain embodiments of the invention, the warming blanket may be used as a reflective and warming layer to reduce heat loss from a human body. In this regard, the warming blanket may be provided in the form of gowns, facemasks, sterilization wraps, head coverings, heating pads, surgical drape, medical warming blanket, and outing warming blanket applications with high reflectivity, good flexibility, enough pliability and breathability. For example, during cold weather in the wild, wrapping a warming blanket around a user's body can help prevent loss of emitted heat and reduce body heat loss.

In accordance with certain embodiments of the invention, the TCL, and/or, the PAL and/or the warming blanket may comprise a desirable level of flexibility (e.g., as measured by Handle-O-Meter) to provide sufficient drapeability and/or wrapability (e.g., wrapped around a user) and/or desirable breathability (e.g., allow vapors to travel through the warming blanket and out the other side of the warming blanket) and/or desirable level of liquid penetration resistance as measured by hydrostatic head.

The terms "substantial" or "substantially" may encompass the whole amount as specified, according to certain embodiments of the invention, or largely but not the whole amount specified (e.g., 95%, 96%, 97%, 98%, or 99% of the whole amount specified) according to other embodiments of the invention.

The terms "polymer" or "polymeric", as used interchangeably herein, may comprise homopolymers, copolymers, such as, for example, block, graft, random, and alternating copolymers, terpolymers, etc., and blends and modifications thereof. Furthermore, unless otherwise specifically limited, the term "polymer" or "polymeric" shall include all possible structural isomers; stereoisomers including, without limitation, geometric isomers, optical isomers or enantionmers; and/or any chiral molecular configuration of such polymer or polymeric material. These configurations include, but are not limited to, isotactic, syndiotactic, and atactic configurations of such polymer or polymeric material. The term "polymer" or "polymeric" shall also include polymers made from various catalyst systems including, without limitation, the Ziegler-Natta catalyst system and the metallocene/single-site catalyst system. The term "polymer" or "polymeric" shall also include, in according to certain embodiments of the invention, polymers produced by fermentation process or biosourced.

The terms "nonwoven" and "nonwoven web", as used herein, may comprise a web having a structure of individual fibers, filaments, and/or threads that are interlaid but not in an identifiable repeating manner as in a knitted or woven fabric. Nonwoven fabrics or webs, according to certain embodiments of the invention, may be formed by any process conventionally known in the art such as, for example, meltblowing processes, spunbonding processes, needle-punching, hydroentangling, air-laid, and bonded carded web processes. A "nonwoven web", as used herein, may comprise a plurality of individual fibers that have not been subjected to a consolidating process.

The terms "fabric" and "nonwoven fabric", as used herein, may comprise a web of fibers in which a plurality of the fibers are mechanically entangled or interconnected, fused together, and/or chemically bonded together. For example, a nonwoven web of individually laid fibers may be subjected to a bonding or consolidation process to bond at least a portion of the individually fibers together to form a coherent (e.g., united) web of interconnected fibers.

The term "consolidated" and "consolidation", as used herein, may comprise the bringing together of at least a portion of the fibers of a nonwoven web into closer proximity or attachment there-between (e.g., thermally fused together, chemically bonded together, and/or mechanically entangled together) to form a bonding site, or bonding sites, which function to increase the resistance to external forces (e.g., abrasion and tensile forces), as compared to the unconsolidated web. The bonding site or bonding sites, for example, may comprise a discrete or localized region of the web material that has been softened or melted and optionally subsequently or simultaneously compressed to form a discrete or localized deformation in the web material. Furthermore, the term "consolidated" may comprise an entire nonwoven web that has been processed such that at least a portion of the fibers are brought into closer proximity or attachment there-between (e.g., thermally fused together, chemically bonded together, and/or mechanically entangled together), such as by thermal bonding or mechanical entanglement (e.g., hydroentanglement) as merely a few examples. Such a web may be considered a "consolidated nonwoven", "nonwoven fabric" or simply as a "fabric" according to certain embodiments of the invention.

The term "staple fiber", as used herein, may comprise a cut fiber from a filament. In accordance with certain embodiments, any type of filament material may be used to form staple fibers. For example, staple fibers may be formed from polymeric fibers, and/or elastomeric fibers. Non-limiting examples of materials may comprise polyolefins (e.g., a polypropylene or polypropylene-containing copolymer), polyethylene terephthalate, and polyamides. The average length of staple fibers may comprise, by way of example only, from about 2 centimeter to about 15 centimeter.

The term "spunbond", as used herein, may comprise fibers which are formed by extruding molten thermoplastic material as filaments from a plurality of fine, usually circular, capillaries of a spinneret with the diameter of the extruded filaments then being rapidly reduced. According to an embodiment of the invention, spunbond fibers are generally not tacky when they are deposited onto a collecting surface and may be generally continuous as disclosed and described herein. It is noted that the spunbond used in certain composites of the invention may include a nonwoven described in the literature as SPINLACE^{®}. Spunbond fibers, for example, may comprises continuous fibers.

As used herein, the term "continuous fibers" refers to fibers which are not cut from their original length prior to being formed into a nonwoven web or nonwoven fabric. Continuous fibers may have average lengths ranging from greater than about 15 centimeters to more than one meter, and up to the length of the web or fabric being formed. For example, a continuous fiber, as used herein, may comprise a fiber in which the length of the fiber is at least 1,000 times larger than the average diameter of the fiber, such as the length of the fiber being at least about 5,000, 10,000, 50,000, or 100,000 times larger than the average diameter of the fiber.

The term "meltblown", as used herein, may comprise fibers formed by extruding a molten thermoplastic material through a plurality of fine die capillaries as molten threads or filaments into converging high velocity, usually hot, gas (e.g. air) streams which attenuate the filaments of molten thermoplastic material to reduce their diameter, which may be to microfiber diameter, according to certain embodiments of the invention. According to an embodiment of the invention, the die capillaries may be circular. Thereafter, the meltblown fibers are carried by the high velocity gas stream and are deposited on a collecting surface to form a web of randomly disbursed meltblown fibers. Meltblown fibers may comprise microfibers which may be continuous or discontinuous and are generally tacky when deposited onto a collecting surface. Meltblown fibers, however, are shorter in length than those of spunbond fibers.

As used herein, the term "monolithic" film may comprise any film that is continuous and substantially free or free of pores (e.g., devoid of pores). In certain alternative embodiments of the invention, a "monolithic" film may comprise fewer pore structures than would otherwise be found in a microporous film. According to certain non-limiting example embodiments of the invention, a monolithic film may act as a barrier to liquids and particulate matter but allow water vapor to pass through. In addition, without intending to be bound by theory, by achieving and maintaining high breathability, it is possible to provide an article that is more comfortable to wear because the migration of water vapor through the laminate helps reduce and/or limit discomfort resulting from excess moisture trapped against the skin. A "monolithic" film, for example, may comprise a highly breathable polymer.

The term "highly breathable polymer", as used herein, may comprise any polymer or elastomer that is selectively permeable to water vapor but substantially impermeable to liquid water and that can form a breathable film, for example, in which the polymer is capable of absorbing and desorbing water vapor and providing a barrier to aqueous fluids (e.g., water, blood, etc.). For example, a highly breathable polymer can absorb water vapor from one side of a film and release it to the other side of film, thereby allowing the water vapor to be transported through the film. As the highly breathable polymer can impart breathability to films, films formed from such polymers do not need to include pores (e.g., monolithic film). According to certain embodiments of the invention, "highly breathable polymer" may comprise any thermoplastic polymer or elastomer having a moisture vapor transmission rate (MVTR) of at least 500 g/m²/day when formed into a film. According to certain embodiments of the invention, "highly breathable polymer" may comprise any thermoplastic polymer or elastomer having a MVTR of at least 750 g/m²/day or of at least 1000 g/m²/day when formed into a film, such as a film having, for example, a thickness of about 25 microns or less. According to certain embodiments of the invention, highly breathable polymers may comprise, for example, any one or combination of a polyether block amide copolymer (e.g., PEBAX^{®} from Arkema Group), polyester block amide copolymer, copolyester thermoplastic elastomer (e.g., ARNITEL^{®} from DSM Engineering Plastics, HYTREL^{®} from E.I. DuPont de Nemours and Company), or thermoplastic urethane elastomer (TPU).

The term "microporous" film, as used herein, may comprise a polymeric film layer hiving a plurality of micropores dispersed throughout a body of the film. Microporous films, for example, may generally be produced by dispersing finely divided particles of a non-hygroscopic filler material, such as an inorganic salt (e.g., calcium carbonate), into a suitable polymer followed by forming a film of the filled polymer and stretching the film to provide good porosity and water vapor absorption or transmission. For example, microporous film breathability may be dependent on the formation of a tortuous porous path throughout the film via the stretching of the filler impregnated film to impart the desired porosity (e.g., pore formation). Furthermore, the barrier properties of such microporous films are affected by the surface tension of the liquid to which they are exposed (e.g., they are more easily penetrated by isopropyl alcohol than by water), and they transmit odor more easily than solid films (e.g., monolithic films).

The term "layer", as used herein, may comprise a generally recognizable combination of similar material types and/or functions existing in the X-Y plane.

All whole number end points disclosed herein that can create a smaller range within a given range disclosed herein are within the scope of certain embodiments of the invention. By way of example, a disclosure of from about 10 to about 15 includes the disclosure of intermediate ranges, for example, of: from about 10 to about 11; from about 10 to about 12; from about 13 to about 15; from about 14 to about 15; etc. Moreover, all single decimal (e.g., numbers reported to the nearest tenth) end points that can create a smaller range within a given range disclosed herein are within the scope of certain embodiments of the invention. By way of example, a disclosure of from about 1.5 to about 2.0 includes the disclosure of intermediate ranges, for example, of: from about 1.5 to about 1.6; from about 1.5 to about 1.7; from about 1.7 to about 1.8; etc.

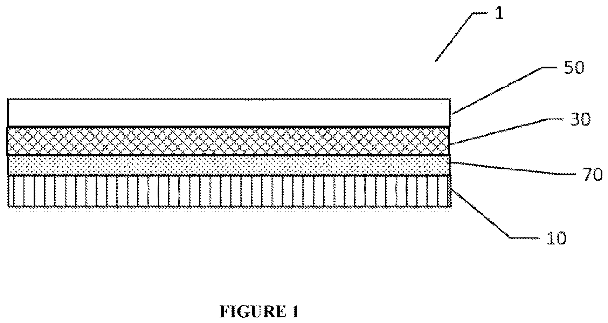

In one aspect, the present invention provides a warming blanket, such as for use outside and/or during the nighttime, including (i) a perspiration absorptive layer (PAL); (ii) a metal coating layer (MCL); and (iii) a transparent coating layer (TCL); wherein the MCL is located directly or indirectly between the PAL and the TCL. Figure 1, for instance, illustrates a warming blanket 1 including a PAL 10, a MCL 30, and a TCL 50, in which the MCL is located between the PAL and the TCL. As shown in Figure 1, the TCL 50 may be adjacent and in contact with the MCL, while a first adhesive layer 70 may be disposed between and bond the PAL to the MCL.

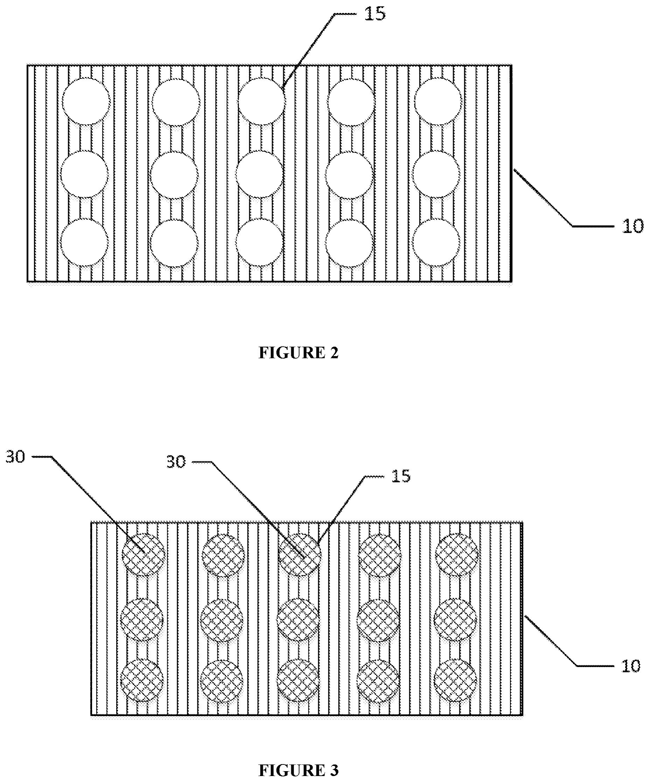

In accordance with certain embodiments of the invention, the PAL may comprise a woven fabric or a nonwoven fabric. As noted above, the PAL may comprise a plurality of through-holes formed through a total thickness of the PAL in a z-direction that is perpendicular to an x-y plane of the PAL. Figure 2, for instance, illustrates a PAL 10 including a plurality of through-holes 15 that extend completely through the entire thickness of the PAL. Meanwhile, Figure 3 illustrates the PAL 10 of Figure 2 overlying a MCL 30 that is visible through the plurality of through-holes 15, in accordance with certain embodiments of the invention.

The plurality of through-holes, in accordance with certain embodiments of the invention, may have an average individual open area from about 1 mm² to about 100 mm², such as at least about any of the following: 1, 3, 5, 8, 10, 15, 20, 25, 30, 35, 40, 45, and 50 mm², and/or at most about any of the following: 100, 95, 90, 85, 80, 75, 70, 65, 60, 55, and 50 mm². In accordance with certain embodiments of the invention, the PAL may include particularly smaller through-holes similar to that of a cross-stitching fabric (e.g., an average individual open area near the lower end of the ranges set forth above). Additionally or alternatively, the PAL may include through-holes having a more macroscopic nature (e.g., an average individual open area near the upper end of the ranges set forth above), in which the through-holes may be formed or cut-out after formation of the fabric. In certain embodiments of the invention, the PAL may comprises a hydroentangled nonwoven fabric, in which the through-holes may have a more macroscopic nature that were formed during a hydroentanglement operation. Additionally or alternatively, the plurality of through-holes may define a total open area from about 10 to about 80%, such as at least about any of the following: 10, 15, 20, 25, 30, 35, 40, 45, and 50%, and/or at most about any of the following: 80, 75, 70, 65, 60, 55, and 50% (e.g., 40% to 60%).

In accordance with certain embodiments of the invention, the PAL may comprise a gridding fabric, such as a woven gridding fabric or a nonwoven gridding fabric. Additionally or alternatively, the PAL may comprise one or more spunbond layers, one or more meltblown layers, one or more cellulose-containing layers, one or more needlepunched layers, one or more hydroentangled layers, one or more carded staple fiber layers, one or more air-laid layers, one or more sub-micron layers, or any combinations thereof. Additionally or alternatively, the PAL may comprise a synthetic polymer, such as one or more polyolefins, one or more polyesters, one or more polyamides, or any combination thereof. Additionally or alternatively, the PAL may comprise a natural cellulosic material, a synthetic cellulosic material, or any combination thereof, such as cotton, pulp, viscose, and rayon. Additionally or alternatively, the PAL may comprise a plurality of superabsorbent polymer (SAP) components, such as beads or particulates, embedded within a body portion of the PAL. For example, the SAP components may be housed or entangled within a plurality of the fibers (e.g., synthetic and/or cellulosic). Additionally or alternatively, the PAL may be provided as a nonwoven web (e.g., non-consolidated) or as a nonwoven fabric that has been consolidated by any means disclosed herein. For example, the PAL may be consolidated by thermal calendering, ultrasonic bonding, mechanical bonding (e.g., hydroentangling), chemical bonding, or any combination thereof).

In accordance with certain embodiments of the invention, the PAL may comprise a spunbond-meltblown-spunbond structure or a spunbond-cellulose-spunbond structure. The PAL, in accordance with certain embodiments of the invention, may comprise a hydroentangled composite formed from a first spunbond layer, a first cellulose-containing layer, and a second spunbond layer. For example, the plurality of through-holes of the PAL may be formed during a hydroentanglement operation.

In accordance with certain embodiments of the invention, the PAL may have a basis weight from 5 to about 500 gsm, such as at least about any of the following: 5, 6, 8, 10, 12, 15, 25, 50, 75, 100, 150, 200, and 250 gsm, and/or at most about any of the following: 500, 450, 400, 350, 300, and 250 gsm.

In accordance with certain embodiments of the invention, the warming blanket includes a first adhesive layer located between and bonding the PAL and the MCL. The first adhesive layer, for example, may comprise a first discontinuous pattern, in which the first discontinuous pattern comprises a first plurality of discrete islands of adhesive surrounded by regions devoid of adhesive. Alternatively, the first adhesive layer may comprise a first discontinuous pattern, in which the first discontinuous pattern comprises a first plurality of discrete islands that are devoid of adhesive and surrounded by regions of adhesive. Alternatively, the first adhesive layer may comprise a first discontinuous pattern, in which the first discontinuous pattern comprises a first plurality of separate and distinct lines of adhesive, and wherein the first plurality of separate and distinct lines of adhesive may be straight, arcuate, or have a zig-zag configuration.

The first discontinuous pattern, in accordance with certain embodiments of the invention, may comprise regions that are devoid of adhesive that are aligned, at least partially, with the plurality of through-holes of the PAL. For example, the first discontinuous pattern may overlap no more than about 50% of the total open area of the PAL, such as at least about any of the following: 0, 3, 5, 8, 10, 12, 15, 18, 20, 22, and 25%, and/or at most about any of the following: 50, 45, 40, 35, 30, 28, 26, and 25%.

In accordance with certain embodiments of the invention, the first adhesive layer may have a basis weight from about 0.2 to about 5 gsm, such as at least about any of the following: 0.25, 0.5, 0,75, 1, 1.5, 2 and 2.5 gsm, and/or at most about any of the following: 5, 4, 3, and 2.5 gsm. Additionally or alternatively, the first adhesive layer, in accordance with certain embodiments of the invention, may comprise a moisture-proof pressure sensitive adhesive, an acrylic holt melt adhesive, or combinations thereof.

In accordance with certain embodiments of the invention, the MCL may comprise a highly reflective metal or highly reflective metal alloy. For example, the highly reflective metal or highly reflective metal alloy may reflect at least about 80% of electromagnetic radiation across all wavelengths from about 1 to about 20 microns, such as across all wavelengths from about 8 to about 15 microns; or such as at least about 85%, or at least about 90%, or at least about 95% of electromagnetic radiation across all wavelengths from about 1 to about 20 microns, such as across all wavelengths from about 8 to about 15 microns. Additionally or alternatively, the highly reflective metal or highly reflective metal alloy may comprise aluminum or an alloy thereof, gold or an alloy thereof, copper or an alloy thereof, silver or an alloy thereof, or any combination thereof. Additionally or alternatively, the MCL may have an average thickness from about 100 nm to about 1,000 nm, such as at least about any of the following: 100, 200, 300, 400, and 500 nm, and/or at most about any of the following: 1000, 900, 800, 700, 600, and 500 nm. Additionally or alternatively, the MCL may have been formed by a vacuum coating method, such as by thermal evaporation, E-beam evaporation, sputtering, arc ion plating, plasma enhanced chemical vapor deposition, or atomic layer deposition.

In accordance with certain embodiments of the invention, the TCL may be directly adjacent the MCL. In this regard, the TCL may be provided and formed, while the MCL may be deposited or otherwise formed directly onto the TCL. The TCL, in accordance with certain embodiments of the invention, may be at least 75% transparent, such as at least 80%, 85%, 90%, 95%, or 99% transparent, to electromagnetic radiation across all wavelengths from about 0.1 to about 0.4 microns. Additionally or alternatively, the TCL may be at least 75% transparent, such as at least 80%, 85%, 90%, 95%, or 99% transparent, to electromagnetic radiation across all wavelengths from about 0.4 to about 0.7 microns. Additionally or alternatively, the TCL may be at least 75% transparent, such as at least 80%, 85%, 90%, 95%, or 99% transparent, to electromagnetic radiation across all wavelengths from about 0.7 to about 1000 microns.

The TCL, in accordance with certain embodiments of the invention, may comprise a polypropylene, a polyethylene, a polyester, such as a polyethylene terephthalate, a thermoplastic elastomer, a thermoplastic polyurethane, a polybutylene terephthalate, polybutylene adipate terephthalate, a polybutyrate, a polylactic acid, or any combinations thereof. By way of example only, the TCL may comprise a polyethylene film having a thickness from 0.10 - 0.12 mm, in which sunlight is around 90% transparent. Additionally or alternatively, the TCL may comprise an anti-reflective coating and defines a first outermost surface of the warming blanket. In accordance with certain embodiments of the invention, the anti-reflective coating, for example, may be a single layer or multiple layers depending on the particular material or materials of construction and transparency desired. By way of example only, the TCL may include a nano-array coating, uniform continuous coating, or a mesoporous structure coating. The coating method, by way of example only, may be an evaporation process, a sputtering process, a roll coating process, as well as other suitable processes for providing a coating to form the TCL. Additionally or alternatively, the TCL may have a thickness from about 5 to about 150 microns, such as at least about any of the following: 5, 10, 15, 20, 25, 30, 40, 50, 60, 70, and 75 microns, and/or at most about any of the following: 150, 125, 100, 90, 80, and 75 microns.

In accordance with certain embodiments of the invention, the TCL may be a film comprising a single layer microporous film or a single layer monolithic film. Alternatively, the film may comprise a multilayer film including one or more microporous films and/or one or more monolithic films.

In accordance with certain embodiments of the invention, the TCL may have a moisture vapor transmission rate (MVTR) of at least about 25 g/m² per 24 hours as determined by ASTM E96D, such as at least about any of the following: 25, 50, 75, 100, 125, 150, 175, and 200 g/m² per 24 hours as determined by ASTM E96D, and/or at most about any of the following: 500, 450, 400, 350, 300, 275, 250, 225, and 200 g/m² per 24 hours as determined by ASTM E96D. Additionally or alternatively, the TCL may have a hydrostatic head (HSH) of at least about 50 mbar as determined by AATCC 127 (60 mbar/min), such as at least about any of the following: 50, 60, 75, 80, 100, and 125 mbar as determined by AATCC 127 (60 mbar/min), and/or at most about any of the following: 200, 175, 150, and 125 mbar as determined by AATCC 127 (60 mbar/min).

In accordance with certain embodiments of the invention, the warming blanket may have a moisture vapor transmission rate (MVTR) of at least about 25 g/m² per 24 hours as determined by ASTM E96D, such as at least about any of the following: 25, 50, 75, 100, 125, 150, 175, and 200 g/m² per 24 hours as determined by ASTM E96D, and/or at most about any of the following: 500, 450, 400, 350, 300, 275, 250, 225, and 200 g/m² per 24 hours as determined by ASTM E96D. Additionally or alternatively, the warming blanket may have a hydrostatic head (HSH) of at least about 50 mbar as determined by AATCC 127 (60 mbar/min), such as at least about any of the following: 50, 60, 75, 80, 100, and 125 mbar as determined by AATCC 127 (60 mbar/min), and/or at most about any of the following: 200, 175, 150, and 125 mbar as determined by AATCC 127 (60 mbar/min).

In another aspect, the present invention provides a method of producing a method of producing a warming blanket, such as those described and disclosed herein. The method may comprise the following: (i) providing a transparent coating layer (TCL); (ii) depositing a metal coating layer (MCL) directly onto the TCL; (iii) providing or forming a perspiration absorptive layer (PAL), which may include a plurality of through-holes as described above; and (iv) bonding the PAL to the MCL to provide the warming blanket, such as those described and disclosed herein.

In accordance with certain embodiments of the invention, the step of bonding the PAL to the MCL may comprise adhesively bonding the PAL directly to the MCL via a first adhesive layer, as noted above. Additionally or alternatively, the first adhesive layer may be deposited onto the PAL, followed by lamination of the PAL to the MCL, in which the first adhesive layer is located between and adjacent the PAL and the MCL. Additionally or alternatively, the first adhesive layer may be deposited onto the MCL, followed by lamination of the PAL and the MCL, in which the first adhesive layer is located between and adjacent the PAL and the MCL. The first adhesive layer, as described above, may comprise a discontinuous pattern.

### Non-Limiting Example Embodiments

The following example embodiments are for illustrative purposes only and highlight that each of the features described in this application can be interchanged with each other in a variety of different manners or configurations.

Example 1: A warming blanket, comprising: (i) a perspiration absorptive layer (PAL); (ii) a metal coating layer (MCL); and (iii) a transparent coating layer (TCL); wherein the MCL is located directly or indirectly between the PAL and the TCL.

Example 2: The warming blanket of example 1, wherein the PAL comprises a woven fabric or a nonwoven fabric.

Example 3: The warming blanket of examples 1-2, wherein the PAL comprises a plurality of through-holes formed through a total thickness of the PAL in a z-direction that is perpendicular to an x-y plane of the PAL.

Example 4: The warming blanket of example 3, wherein the plurality of through-holes have an average individual open are from about 1 mm² to about 100 mm², such as at least about any of the following: 1, 3, 5, 8, 10, 15, 20, 25, 30, 35, 40, 45, and 50 mm², and/or at most about any of the following: 100, 95, 90, 85, 80, 75, 70, 65, 60, 55, and 50 mm².

Example 5: The warming blanket of examples 3-4, wherein the plurality of through-holes define a total open area from about 10 to about 80%, such as at least about any of the following" 10, 15, 20, 25, 30, 35, 40, 45, and 50%, and/or at most about any of the following: 80, 75, 70, 65, 60, 55, and 50%. (e.g., 40% to 60%).

Example 6: The warming blanket of examples 1-5, wherein the PAL comprises a gridding fabric, such as a woven gridding fabric or a nonwoven gridding fabric.

Example 7: The warming blanket of examples 1-6, wherein the PAL comprises one or more spunbond layers, one or more meltblown layers, one or more cellulose-containing layers, one or more needlepunched layers, one or more hydroentangled layers, one or more carded layer, one or more sub-micron layers, or any combinations thereof; and wherein the PAL comprises a synthetic polymer, such as one or more polyolefins, one or more polyesters, one or more polyamides, a natural cellulosic material, a synthetic cellulosic material, or any combination thereof.

Example 8: The warming blanket of example 7, wherein the PAL comprises a spunbond-meltblown-spunbond structure.

Example 9: The warming blanket of example 7, wherein the PAL comprises a spunbond-cellulose-spunbond structure.

Example 10: The warming blanket of example 9, wherein the PAL comprises a hydroentangled composite formed from a first spunbond layer, a first cellulose-containing layer, and a second spunbond layer.

Example 11: The warming blanket of examples 1-10, wherein the PAL comprises a plurality of superabsorbent polymer (SAP) components, such as beads or particulates, embedded within a body portion of the PAL.

Example 12: The warming blanket of examples 1-11, wherein the PAL has a basis weight from 5 to about 500 gsm, such as at least about any of the following: 5, 6, 8, 10, 12, 15, 25, 50, 75, 100, 150, 200, and 250 gsm, and/or at most about any of the following: 500, 450, 400, 350, 300, and 250 gsm.

Example 13: The warming blanket of examples 1-12, further comprising a first adhesive layer located between and bonding the PAL and the MCL.

Example 14: The warming blanket of example 13, wherein the first adhesive layer comprises a first discontinuous pattern, wherein the first discontinuous pattern comprises a first plurality of discrete islands of adhesive surrounded by regions devoid of adhesive.

Example 15: The warming blanket of example 13, wherein the first adhesive layer comprises a first discontinuous pattern, wherein the first discontinuous pattern comprises a first plurality of discrete islands that are devoid of adhesive and surrounded by regions of adhesive.

Example 16: The warming blanket of example 13, wherein the first adhesive layer comprises a first discontinuous pattern, wherein the first discontinuous pattern comprises a first plurality of separate and distinct lines of adhesive, and wherein the first plurality of separate and distinct lines of adhesive may be straight, arcuate, or have a zig-zag configuration.

Example 17: The warming blanket of examples 14-16, wherein the first discontinuous pattern comprises regions that are devoid of adhesive that are aligned with the plurality of through-holes of the PAL.

Example 18: The warming blanket of examples 14-17, wherein the first discontinuous pattern overlaps no more than about 50% of the total open area of the PAL, such as at least about any of the following: 0, 3, 5, 8, 10, 12, 15, 18, 20, 22, and 25%, and/or at most about any of the following: 50, 45, 40, 35, 30, 28, 26, and 25%.

Example 19: The warming blanket of examples 13-18, wherein the first adhesive layer has a basis weight from about 0.2 to about 5 gsm, such as at least about any of the following: 0.25, 0.5, 0,75, 1, 1.5, 2 and 2.5 gsm, and/or at most about any of the following: 5, 4, 3, and 2.5 gsm.

Example 20: The warming blanket of examples 13-19, wherein the first adhesive layer comprises a moisture-proof pressure sensitive adhesive, an acrylic holt melt adhesive, or combinations thereof.

Example 21: The warming blanket of examples 1-20, wherein the MCL comprises a highly reflective metal or highly reflective metal alloy.

Example 22: The warming blanket of example 21, wherein the highly reflective metal or highly reflective metal alloy reflects at least about 80% of electromagnetic radiation across all wavelengths from about 1 to about 20 microns, such as across all wavelengths from about 8 to about 15 microns; or such as at least about 85%, or at least about 90%, or at least about 95% of electromagnetic radiation across all wavelengths from about 1 to about 20 microns, such as across all wavelengths from about 8 to about 15 microns.

Example 23: The warming blanket of examples 21-22, wherein the highly reflective metal or highly reflective metal alloy comprises aluminum or an alloy thereof, gold or an alloy thereof, copper or an alloy thereof, silver or an alloy thereof, or any combination thereof.

Example 24: The warming blanket of examples 21-23, wherein the MCL has an average thickness from about 100 nm to about 1,000 nm, such as at least about any of the following: 100, 200, 300, 400, and 500 nm, and/or at most about any of the following: 1000, 900, 800, 700, 600, and 500 nm.

Example 25: The warming blanket of examples 21-24, wherein the MCL has been formed by a vacuum coating method, such as by thermal evaporation, E-beam evaporation, sputtering, arc ion plating, plasma enhanced chemical vapor deposition, or atomic layer deposition.

Example 26: The warming blanket of examples 1-25, wherein the TCL is directly adjacent the MCL, and optionally comprises a polyethylene film.

Example 27: The warming blanket of examples 1-26, wherein the TCL is at least 75% transparent, such as at least 80%, 85%, 90%, 95%, or 99% transparent, to electromagnetic radiation across all wavelengths from about 0.1 to about 0.4 microns.

Example 28: The warming blanket of examples 1-27, wherein the TCL is at least 75% transparent, such as at least 80%, 85%, 90%, 95%, or 99% transparent, to electromagnetic radiation across all wavelengths from about 0.4 to about 0.7 microns.

Example 29: The warming blanket of examples 1-28, wherein the TCL is at least 75% transparent, such as at least 80%, 85%, 90%, 95%, or 99% transparent, to electromagnetic radiation across all wavelengths from about 0.7 to about 1000 microns.

Example 30: The warming blanket of examples 1-29, wherein the TCL comprises a polypropylene, a polyethylene, a polyester, such as a polyethylene terephthalate, a thermoplastic elastomer, a thermoplastic polyurethane, a polybutylene terephthalate, polybutylene adipate terephthalate, a polybutyrate, a polylactic acid, or any combinations thereof.

Example 31: The warming blanket of examples 1-30, wherein the TCL comprises an anti-reflective coating and defines a first outermost surface of the warming blanket.

Example 32: The warming blanket of examples 1-31, wherein the TCL has a thickness from about 5 to about 150 microns, such as at least about any of the following: 5, 10, 15, 20, 25, 30, 40, 50, 60, 70, and 75 microns, and/or at most about any of the following: 150, 125, 100, 90, 80, and 75 microns.

Example 33: The warming blanket of examples 1-32, wherein the TCL is a film comprising a single layer microporous film or a single layer monolithic film.

Example 34: The warming blanket of example 33, wherein the film comprises a multilayer film including one or more microporous films and/or one or more monolithic films.

Example 35: The warming blanket of examples 1-34, wherein the TCL has a moisture vapor transmission rate (MVTR) of at least about 25 g/m² per 24 hours as determined by ASTM E96D, such as at least about any of the following: 25, 50, 75, 100, 125, 150, 175, and 200 g/m² per 24 hours as determined by ASTM E96D, and/or at most about any of the following: 500, 450, 400, 350, 300, 275, 250, 225, and 200 g/m² per 24 hours as determined by ASTM E96D.

Example 36: The warming blanket of examples 1-35, wherein the TCL has a hydrostatic head (HSH) of at least about 50 mbar as determined by AATCC 127 (60 mbar/min), such as at least about any of the following: 50, 60, 75, 80, 100, and 125 mbar as determined by AATCC 127 (60 mbar/min), and/or at most about any of the following: 200, 175, 150, and 125 mbar as determined by AATCC 127 (60 mbar/min).

Example 37: The warming blanket of examples 1-36, wherein the warming blanket has a moisture vapor transmission rate (MVTR) of at least about 25 g/m² per 24 hours as determined by ASTM E96D, such as at least about any of the following: 25, 50, 75, 100, 125, 150, 175, and 200 g/m² per 24 hours as determined by ASTM E96D, and/or at most about any of the following: 500, 450, 400, 350, 300, 275, 250, 225, and 200 g/m² per 24 hours as determined by ASTM E96D.

Example 38: The warming blanket of examples 1-37, wherein the warming blanket has a hydrostatic head (HSH) of at least about 50 mbar as determined by AATCC 127 (60 mbar/min), such as at least about any of the following: 50, 60, 75, 80, 100, and 125 mbar as determined by AATCC 127 (60 mbar/min), and/or at most about any of the following: 200, 175, 150, and 125 mbar as determined by AATCC 127 (60 mbar/min).

Example 39: A method of producing a warming blanket, such as those according to examples 1-38, comprising: (i) providing a transparent coating layer (TCL); (ii) depositing a metal coating layer (MCL) directly onto the TCL; (iii) providing or forming a perspiration absorptive layer (PAL); and (iv) bonding the PAL to the MCL to provide the warming blanket.

Example 40: The method of example 39, wherein bonding the PAL to the MCL comprises adhesively bonding the PAL directly to the MCL via a first adhesive layer.

Example 41: The method of example 40, wherein the first adhesive layer is deposited onto the PAL, followed by lamination of the PAL to the MCL; wherein the first adhesive layer is located between and adjacent the PAL and the MCL.

Example 42: The method of example 40, wherein the first adhesive layer is deposited onto the MCL, followed by lamination of the PAL and the MCL; wherein the first adhesive layer is located between and adjacent the PAL and the MCL.

These and other modifications and variations to the invention may be practiced by those of ordinary skill in the art without departing from the spirit and scope of the invention, which is more particularly set forth in the appended claims. In addition, it should be understood that aspects of the various embodiments may be interchanged in whole or in part. Furthermore, those of ordinary skill in the art will appreciate that the foregoing description is by way of example only, and it is not intended to limit the invention as further described in such appended claims. Therefore, the spirit and scope of the appended claims should not be limited to the exemplary description of the versions contained herein.

## Claims

1. A warming blanket, comprising:
(i) a perspiration absorptive layer (PAL) comprising a woven fabric or a nonwoven fabric, wherein the PAL comprises a plurality of through-holes formed through a total thickness of the PAL in a z-direction that is perpendicular to an x-y plane of the PAL;
(ii) a metal coating layer (MCL); and
(iii) a transparent coating layer (TCL) comprising a breathable film; wherein the MCL is located directly or indirectly between the PAL and the TCL.

2. The warming blanket of claim 1, the breathable film comprises a microporous film, a monolithic film, or a combination thereof.

3. The warming blanket of claim 2, wherein the TCL has a moisture vapor transmission rate (MVTR) of at least about 25 g/m² per 24 hours as determined by ASTM E96D .

4. The warming blanket according to any one of claims 1-3, wherein the TCL has a thickness from 5 to 150 microns.

5. The warming blanket of claim 4, wherein (i) the plurality of through-holes have an average individual open are from about 1 mm² to about 100 mm²; (ii) the plurality of through-holes define a total open area from about 10 to about 80%; or (iii) both (i) and (ii).

6. The warming blanket of claim 5, further comprising a first adhesive layer located between and bonding the PAL and the MCL, wherein the first adhesive layer comprises a first discontinuous pattern including regions that are devoid of adhesive that are at least partially aligned with the plurality of through-holes of the PAL.

7. The warming blanket of claim 6, wherein the first discontinuous pattern overlaps no more than about 50% of the total open area of the PAL.

8. The warming blanket of claim 1, wherein the PAL has a basis weight from 5 to 500 gsm.

9. The warming blanket of claim 1, wherein the MCL comprises a highly reflective metal or highly reflective metal alloy, such as aluminum or an alloy thereof, gold or an alloy thereof, copper or an alloy thereof, silver or an alloy thereof, or any combination thereof .

10. The warming blanket of claim 1, wherein the TCL is directly adjacent the MCL.

11. The warming blanket of claim 1, further comprising anti-reflective coating disposed on the TCL and defines a first outermost surface of the warming blanket.

12. The warming blanket of claim 1, wherein the warming blanket has a hydrostatic head (HSH) of at least 50 mbar as determined by AATCC 127 (60 mbar/min).

13. The warming blanket of claim 1, wherein the warming blanket has a moisture vapor transmission rate (MVTR) of at least about 25 g/m² per 24 hours as determined by ASTM E96D.

14. A method of producing a warming blanket according to claim 1, comprising:
(i) providing a transparent coating layer (TCL);
(ii) depositing a metal coating layer (MCL) directly onto the TCL;
(iii) providing or forming a perspiration absorptive layer (PAL); and
(iv) bonding the PAL to the MCL to provide the warming blanket.

15. The method of claim 14, wherein depositing the MCL directly onto the TCL comprises formation of the MCL via a vacuum coating method, such as by thermal evaporation, E-beam evaporation, sputtering, arc ion plating, plasma enhanced chemical vapor deposition, or atomic layer deposition.
